# EUROPEAN PATENT APPLICATION

(11) **EP 3 973 961 A1**
(43) Date of publication of application: **30.03.2022**
(21) Application number: 20306117.1
(22) Date of filing: 29.09.2020
(51) Int. Cl.: A61K 31/445, A61K 31/4545, A61K 31/495, A61P 31/14

(54) **USE OF HYDROXYZINE AND ANTIHISTAMINE ANALOGS FOR TREATING THE COVID-19 DISEASE**

(71) Applicant: ASSISTANCE PUBLIQUE, HOPITAUX DE PARIS, 75004 Paris (FR)
(72) Inventor: HOERTEL, Nicolas, 92130 ISSY LES MOULINEAUX (FR); LIMOSIN, Frédéric, 75015 PARIS (FR); SANCHEZ RICO, Marina Lucia, 92130 ISSY LES MOULINEAUX (FR)
(74) Representative: Regimbeau

(57) **Abstract**

The present invention relates to the therapeutic uses of hydroxyzine and analogs thereof for lowering the risk of death and/or intubation in patients suffering from a viral infection caused by the SARS-CoV-2 (Severe Acute Respiratory Syndrome Coronavirus 2).

## Description

### SUMMARY OF THE INVENTION

The present invention relates to the therapeutic uses of hydroxyzine and analogs thereof for lowering the risk of death and/or intubation in patients suffering from a viral infection caused by the SARS-CoV-2 (Severe Acute Respiratory Syndrome Coronavirus 2).

### BACKGROUND OF THE INVENTION

Global spread of the novel coronavirus SARS-CoV-2, the causative agent of coronavirus disease 2019 (Covid-19), has created an unprecedented infectious disease crisis worldwide. In the current absence of a vaccine or medications with published evidence-based clinical efficacy, the search for an effective treatment for patients with Covid-19 among all available medications is urgently needed.

SARS-CoV-2 belongs to the species *Coronavirus,* in the genus *Betacoronavirus* and family *Coronaviridae.* Coronaviruses are enveloped viruses with a helically symmetrical capsid. They have a single-stranded, positive-sense RNA genome and are capable of infecting cells in birds and mammals. The morphology of the virions is typical, with a halo of protein protuberances ('Spike') which gave them their name of 'crown virus'. The Betacoronaviruses of the greatest clinical importance concerning humans are:
- OC43 and HKU1 of the A lineage,
- SARS-CoV-1 and SARS-CoV-2 of the B lineage, and
- MERS-CoV of the C lineage.

In humans, coronavirus infections can cause respiratory pathologies associated with symptoms similar to the common cold, bronchiolitis and more serious diseases such as the Severe Acute Respiratory Syndrome caused by SARS-CoV-1, which generated an epidemic in 2003, and the Middle Eastern Respiratory Syndrome caused by MERS-CoV, which generated an epidemic in 2012. SARS-CoV-2 is the betacoronavirus causing the coronavirus epidemic of 2019-2020, generating the form of pneumonia known as coronavirus disease 2019 or COVID-19. According to the World Health Organization (WHO), by September 29, 2020, 33 400 000 cases of COVID-19 have been confirmed and 1000 000 patients have died worldwide.

Symptoms of infection with SARS-CoV-2 are roughly similar to those of seasonal influenza infections: they include fever, fatigue, dry cough, shortness of breath, difficult breathing, pneumonia, renal failure, and may lead to death in severe cases.

The severity of clinical signs requires that approximately 20% of patients remain in hospital and 5% require admission to intensive care. The most serious forms are observed in people who are vulnerable because of their age (over 70) or associated diseases such as hypertension, diabetes and/or coronary heart disease.

The majority of treatments currently received by COVID-19 patients are primarily aimed at alleviating the symptoms of fever, cough and dyspnea in order to promote spontaneous recovery. Yet, no efficient treatment has ever been proposed to prevent intubation or death of the patient once they are in a severe stage of the disease.

It is of the most importance to identify therapeutic compounds for treating the COVID-19 disease in order to prevent the risks of death, intubation and hospitalization in severely infected patient. The present invention fulfills this need.

### DESCRIPTION OF THE INVENTION

In this context, the present inventors performed a large (7,345 adult inpatients with COVID-19, including 138 (1.9%) patients receiving hydroxyzine during the hospitalization) multicenter observational retrospective study, and examined the association between hydroxyzine use with the risk of death, ascertained by death certificates, among male and female adult patients (aged 18 to 103 years old of age) who have been admitted to these medical centers with COVID-19, ascertained by a positive reverse-transcriptase-polymerase-chain-reaction (RT-PCR) test from analysis of nasopharyngeal or oropharyngeal swab specimens.

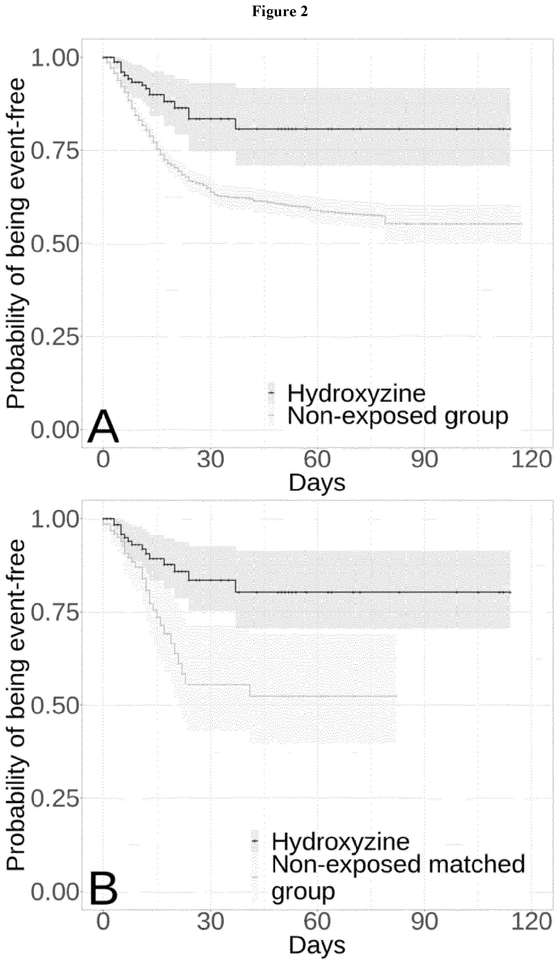

They found that hydroxyzine, prescribed for others indications (including urticaria, allergic rhinitis, hay fever, conjunctivitis, pruritis) and for its tranquilizer and sedative properties, are significantly and substantially associated with reduced risk of death in hospitalized adult patients with Covid-19, independently of patients' characteristics, disease's severity and use of other psychotropic medications (figure 2).

They found that the effect of hydroxyzine on COVID-19 is unlikely to be confounded by its indication, and is not explained by its effects on sleep or anxiety. Instead, the effect of hydroxyzine on COVID-19 is likely to be due to biological anti-inflammatory effects, probably through (i) H1 receptor antagonism and selective inhibitor of peripheral H1 receptors, (ii) inhibition of NF-kB dependent cytokines (such as IL-1, IL-2, IL-6, IL-8, IL-12, TNF-α) and adhesion proteins (such as ICAM-1, VCAM-1 and ECAM-1), (iii) and the inhibition of the release by inflammatory cells of pre-formed mediators, such as histamine and eosinophil proteins, as well as eicosanoid generation and oxygen free radicals production, and possibly through possible direct antiviral anti-SARS-CoV-2 effects. Moreover, hydroxyzine could decrease biological markers of inflammation, such as blood levels of interleukin 6, the neutrophil-to-lymphocyte ratio, and the lymphocyte-to-C-reactive protein ratio, which are associated with increased COVID-19-related mortality. Finally, histamine H1 receptor (H1R) signaling could affect glucocorticoid receptor (GR)-mediated activity, impacting on its transcriptional outcome through a dual regulation of GR activity by the H1R: a potentiation mediated by G-protein βγ subunits and a parallel inhibitory effect mediated by Gaq-PLC pathway ; activation of the H1R by its full agonists as well as inactivation of the Gaq-PLC pathway by H1R inverse agonists could both result in a composite potentiating inflammatory effect ; therefore, antihistamines may synergize with the GR agonist dexamethasone to induce gene transactivation and transrepression in a gene-specific manner.

In this context, the present invention proposes to use inhibitors of peripheral Histamine receptors for treating a viral infection due to a Severe Acute Respiratory Syndrome-related coronavirus (SARS-CoV), in particular SARS-CoV-2, in a patient in need thereof. Said inhibitors can also be efficient for treating patients suffering from a symptomatic COVID-19 disease, advantageously from a strong or severe symptomatic COVID-19 disease. Particularly, said inhibitors can prevent and/or treat acute respiratory distress syndrome associated to said viral infection ; and/or lower the risk of intubation and/or lower the risk of hospitalization and/or eventually increase the survival rate of patients infected by said coronavirus.

There are four histamine receptors which bind histamine as their primary endogenous ligand (namely the H1 receptor, H2 receptor, H3 receptor, and H4 receptor). Although the inventors have gathered data involving anti-H1 compounds, the three other histamine receptors are likely to be involved in the regulation of the COVID-related mortality as well, because of the well-known cross regulation occurring between all these histamine receptors.

In a particular embodiment, the present invention relates to the use of inhibitors of peripheral H2 receptors, said inhibitors being for example chosen in the group consisting of: cimetidine, famotidine, lafutidine, nizatidine, ranitidine, roxatidine, and tiotidine.

In a particular embodiment, the present invention relates to the use of inhibitors of peripheral H3 receptors, said inhibitors being for example chosen in the group consisting of: clobenpropit, ABT-239, Ciproxifan, conessine, A-349,821, and thioperamide.

In a particular embodiment, the present invention relates to the use of inhibitors of peripheral H4 receptors, said inhibitors being for example chosen in the group consisting of: thioperamide, JNJ7777120, and VUF-6002.

In a particular embodiment, the present invention relates to the use of inhibitors of the histidine decarboxylase enzyme, said inhibitors being for example tritoqualine or catechine.

The present invention more precisely relates to the use of inhibitors of peripheral H1 receptors for treating a viral infection due to a Severe Acute Respiratory Syndrome-related coronavirus (SARS-CoV), in particular SARS-CoV-2, in a patient in need thereof. Said inhibitors can also be efficient for treating patients suffering from a symptomatic COVID-19 disease, advantageously from a strong or severe symptomatic COVID-19 disease. Particularly, said inhibitors can prevent and/or treat acute respiratory distress syndrome associated to said viral infection ; and/or lower the risk of intubation and/or lower the risk of hospitalization and/or eventually increase the survival rate of patients infected by said coronavirus.

In a first aspect, the present invention relates to an inhibitor of peripheral H1 receptors for use for treating a viral infection due to a Severe Acute Respiratory Syndrome-related coronavirus (SARS-CoV), in particular SARS-CoV-2, in a patient in need thereof. It also relates to said inhibitor, for its use for treating patients suffering from a symptomatic COVID-19 disease, advantageously from a strong or severe symptomatic COVID-19 disease. It also relates to said inhibitor, for its use for preventing and/or treating acute respiratory distress syndrome associated to said viral infection. Finally, it also relates to said inhibitor, for its use for lowering the risk of intubation and/or for lowering the risk of hospitalization and/or for increasing the survival rate of patients (i.e., decreasing risk of death) infected by said coronavirus.

In other words, the present invention relates to the use of an inhibitor of peripheral H1 receptors for the preparation of a medicament that is intended to treat a symptomatic COVID-19 disease, advantageously from a strong or severe symptomatic COVID-19 disease; or an acute respiratory distress syndrome associated to said viral infection.

The present invention also discloses a method for treating a viral infection due to a Severe Acute Respiratory Syndrome-related coronavirus (SARS-CoV), in particular SARS-CoV-2, in a patient in need thereof, said method comprising administering in said patient a significant amount of an inhibitor of peripheral H1 receptors. It also discloses a method for treating patients suffering from a symptomatic COVID-19 disease, a method for preventing and/or treating acute respiratory distress syndrome associated to said viral infection, and a method for lowering the risk of intubation and/or for increasing the survival rate (i.e., decreasing risk of death) of patients infected by said coronavirus, said methods comprising administering in said patients a significant amount of an inhibitor of peripheral H1 receptors.

As used herein, the term "inhibitor of the invention" stands for "inhibitor of peripheral H1 receptors" that is to be used in the methods and therapeutic applications of the invention. These terms are herein used interchangeably with the expressions "H1 antihistamine compound" or "H1 antihistamine" or "H1 inverse agonist" or "H1 antagonist" or "H1 blocker" or, even more generally, "antihistamine" or "H1 compound". This inhibitor binds to, or inhibits the binding of a ligand to, the H1 histamine receptor, thereby reducing or eliminating the activity of the H1 histamine receptor.

The histamine H1-receptor is a member of the superfamily of G-protein-coupled receptors (GPCRs). Histamine cross links sites on transmembrane domains III and V to stabilize the receptor in its active conformation, thus causing the equilibrium to switch to the position "on". H1 antihistamine, which are not structurally related to histamine, do not antagonize the binding of histamine but bind to different sites on the receptor, to produce the opposite effect. Thus, H1-antihistamines are not receptor antagonists, but inverse agonists of the H1 receptor.

In some embodiments, the inhibitor of the invention inhibits the activity of the H1 receptor by at least 5%, 10%, 20%, 30%, 40%, 50%, 60%, 70%, 80%, 90%, 95% or 100% (i.e., complete loss of activity) relative to a control. In some embodiment, the inhibitor of the invention is capable of binding to an allosteric site of the histamine H1 receptor. In a particular embodiment, the inhibitor of the invention has an IC₅₀ of less than or equal to 500nM, less than or equal to 250nM, less than or equal to 100nM, less than or equal to 50nM, less than or equal to 10nM, less than or equal to 1nM, less than or equal to 0,1nM, less than or equal to 0,01nM, less than or equal to 0,001nM. Said inhibitory effect can be tested by conventional means, such as those disclosed in Diaz Nebreda et al.

H1 antihistamine compounds are widely used in the treatment of urticaria, allergic rhinitis, hay fever, conjunctivitis and pruritis. They work by competitive binding to H1 receptors and inhibiting the action of histamine, a primary mediator of an early-phase allergic inflammation response that also modulates the late-phase response characterized by cellular influx of eosinophils, neutrophils, basophils, mononuclear cells, and T lymphocytes. *In vivo* and *in vitro* studies have also suggested additional anti-inflammatory properties of H1 antihistamines, including both receptor-dependent and receptor-independent mechanisms. The receptor-dependent mechanisms may involve inhibition of NF-kB dependent cytokines (such as IL-1, IL-2, IL-6, IL-8, IL-12, TNF-α) and adhesion proteins (such as ICAM-1, VCAM-1 and ECAM-1). The receptor-independent mechanisms, which require higher drug concentrations, may include the release by inflammatory cells of pre-formed mediators, such as histamine and eosinophil proteins, as well as eicosanoid generation and oxygen free radical production.

The H1 compounds belonging to the first generation of antihistamine compounds that have been disclosed are relatively inexpensive and widely available. They can be classified on the basis of their chemical structure: ethylenediamines (such as mepyramine, chloropyramine, antazoline, tripelennamine), ethanolamines (such as diphenhydramine, carbinoxamine, doxylamine, orphenadrine, bromazine, clemastine, dimenhydrinate), alkylamines (such as pheniramine, chlorphenamine, dexchlorpheniramine, dexbrompheniramine, brompheniramine, triprolidine, dimetindene), piperazines (such as chlorcyclizine, hydroxyzine, buclizine, cetirizine, cinnarizine, cyclizine, etodroxizine, meclizine, and pipoxizine, among others), tricyclics and tetracyclics (such as promethazine, alimemazine, cyproheptadine).

All these first generation H1 compounds share the common structural formula I:

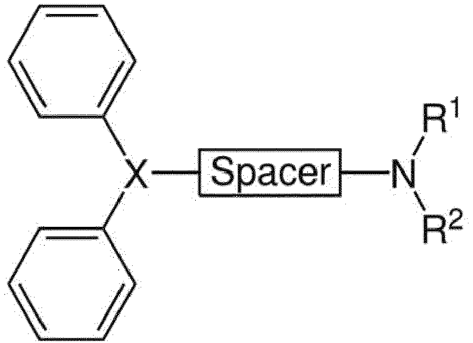

They indeed contain two aromatic rings, connected to a central carbon, nitrogen or CO (X = N, C or CO); a spacer between the central X and the amine (said spacer being usually 2-3 carbons in length, linear, ring, branched, saturated or unsaturated). The amine can be substituted with small alkyl groups, e.g., CH₃, or with longer of cyclic groups. The chirality at X increases both the potency and selectivity of the antihistamine compound for the H1 receptor.

Within the meaning of this invention, "stereoisomers" is intended to designate diastereoisomers or enantiomers. These are therefore optical isomers. Stereoisomers which are not mirror images of one another are thus designated as "diastereoisomers," and stereoisomers which are non-superimposable mirror images are designated as "enantiomers".

Within the meaning of this invention, "conformers" is intended to designate a form of stereoisomers in which the isomers can be interconverted just by rotations about formally single bonds.

In a preferred embodiment, the inhibitor of the invention is of formula I, with X, spacer and the amine as described above.

In a preferred embodiment, the inhibitor of the invention is a first generation antihistamine compound chosen in the group consisting of: mepyramine, chloropyramine, antazoline, tripelennamine, diphenhydramine, carbinoxamine, doxylamine, orphenadrine, bromazine, clemastine, dimenhydrinate, pheniramine, chlorphenamine, dexchlorpheniramine, dexbrompheniramine, brompheniramine, triprolidine, dimetindene, buclizine, cinnarizine, cyclizine, etodroxizine, chlorcyclizine, hydroxyzine, pipoxizine, meclizine, promethazine, alimemazine, cyproheptadine, setastine, or a pharmaceutical salt or solvate thereof. Stereoisomers and conformers thereof are also herein encompassed.

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

In an even more preferred embodiment, the inhibitor of the invention has a diphenyl methyl piperazine structure, for example chosen in the group consisting of: meclizine, buclizine, cinnarizine, cyclizine, cetirizine, etodroxizine, chlorcyclizine, and hydroxyzine, or a pharmaceutical salt or solvate thereof. All other diphenyl methyl piperazine compounds known in the art are herewith encompassed.

In a number of prior art studies, meclizine, buclizine, cinnarizine, cyclizine, cetirizine, etodroxizine, and chlorcyclizine are referred to as "analogs of hydroxyzine". Thus, in other words, the inhibitor of the invention is an analog of hydroxyzine.

Even more preferably, the inhibitor of the invention is hydroxyzine or a pharmaceutical salt or solvate thereof. Stereoisomers and conformers thereof are also herein encompassed.

Hydroxyzine is a member of the diphenylmethylpiperazine class of antihistamines. It is sold under the brand names Atarax and many others (Vistaril, Equipose, Masmoran, Paxistil, Alamon, Aterax, Durrax, Tran-Q, Orgatrax, Quiess, Tranquizine, etc.). Its CAS number is 68-88-2. It is also known as hydroxizine, or hydroxycine. Its chemical formula is 2-[2-[4-[(4-chlorophenyl)-phenylmethyl]piperazin-1-yl]ethoxy]ethanol:

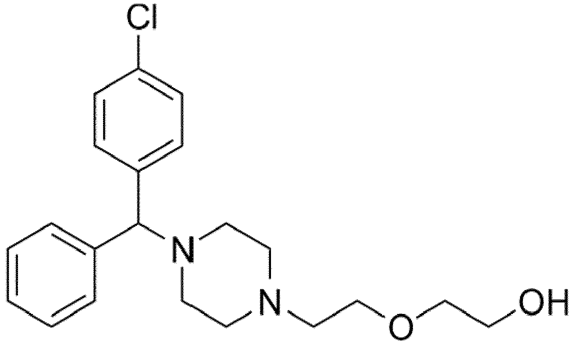

Any salt thereof can be used in the invention. Preferably, said salt can be a pamoate (CAS 10246-75-0) or a hydrochloride or a di-hydrochloride salt (CAS2192-693-1). These salts are commercially available and therefore very easy to acquire and use: Vistaril, Equipose, Masmoran, and Paxistil are preparations of the pamoate salt, while Atarax, Alamon, Aterax, Durrax, Tran-Q, Orgatrax, Quiess, and Tranquizine are of the hydrochloride salt.

Hydroxyzine is preferably administered orally or via intramuscular injection. When given orally, hydroxyzine is rapidly absorbed from the gastrointestinal tract. The effect of hydroxyzine is notable in 30 minutes.

Among first generation antihistamines, hydroxyzine is one of the most prescribed in France. Beyond its antihistamine activity, hydroxyzine is also prescribed as a psychotropic medication for its tranquilizer and sedative properties, as it weakly acts as an antagonist of the serotonin 5-HT2A-receptor, the dopamine D2 receptor, and the α1-adrenergic receptor. Because prior research supports that severe COVID-19 is characterized by an overexuberant inflammatory response and that viral load has been associated with the worsening of symptoms it was hypothesized that the antihistamine compound hydroxyzine could be potentially effective in reducing the risk of death in patients with COVID-19. The results disclosed below show that hydroxyzine, prescribed for others indications (including urticaria, allergic rhinitis, hay fever, conjunctivitis, pruritis) and for its tranquilizer and sedative properties, are significantly and substantially associated with reduced risk of death in hospitalized adult patients with Covid-19, independently of patients' characteristics, disease's severity and use of other psychotropic medications.

The H1 inhibitor of the invention can also be a second-generation H1 antihistamine. These second-generation compounds are newer drugs that are much more selective for peripheral H1 receptors than for H1 receptors of the central nervous system and for cholinergic receptors. This selectivity significantly reduces the occurrence of adverse drug reactions, such as sedation, while still providing effective relief of allergic conditions. The reason for their peripheral selectivity is that most of these compounds are zwitterionic at physiological pH (around pH 7.4). As such, they are very polar, meaning that they are less likely to cross the blood-brain barrier and act mainly outside the central nervous system. Compounds of second generation H1 antihistamine are for example: astemizole, ketotifen, cetirizine, loratadine, rupatadine, mizolastine, acrivastine, ebastine, bilastine, bepotastine, terfenadine, quifenadine, loratadine, desloratadine, azelastine, levocabastine, olopatadine.

In a particular embodiment, the inhibitor of the invention is a second-generation H1 antihistamine chosen in the group consisting of: astemizole, ketotifen, cetirizine, loratadine, desloratadine, rupatadine, mizolastine, acrivastine, ebastine, bilastine, bepotastine, terfenadine, quifenadine, azelastine, levocabastine, olopatadine, or a pharmaceutical salt or solvate thereof. Stereoisomers and conformers thereof are also herein encompassed.

It is known in the art that hydroxyzine is metabolized in the liver into its main metabolite (45%), cetirizine. Cetirizine is indeed formed *in vivo* through oxidation of the alcohol moiety to a carboxylic acid, by the alcohol dehydrogenase. Cetirizine, although less sedating, is non-dialyzable and possesses similar anti-histaminergic properties as hydroxyzine do.

Thus, in another particular embodiment, the inhibitor of the invention is the diphenylmethylpiperazine cetirizine or a pharmaceutical salt or solvate thereof, preferably cetirizine hydrochloride. Stereoisomers and conformers thereof are also herein encompassed.

Cetirizine is marketed under the brand names Alatrol, Alerid, Alzene, Cetirin, Cetzine, Cezin, Cetgel, Cirrus, Histec, Histazine, Humex, Letizen, Okacet (Cipla), Reactine, Razene, Rigix, Sensahist (Oethmann, South Africa), Triz, Zetop, Zirtec, Zirtek, Zodac, Zyllergy, Zynor, Zyrlek, and Zyrtec (Johnson & Johnson), among others.

Cetirizine contains L- and D-stereoisomers. Chemically, levocetirizine is the active L-enantiomer of cetirizine. Thus, in a preferred embodiment, the inhibitor of the in invention is the active L-enantiomer of cetirizine (levocetirizine), or a pharmaceutical salt or solvate thereof, preferably levocetirizine hydrochloride.

Third-generation H1-antihistamines are second-generation antihistamines informally labeled "third-generation" because the active enantiomer (levocetirizine) or metabolite (desloratadine and fexofenadine) derivatives of second-generation drugs are intended to have increased efficacy with fewer adverse drug reactions. Fexofenadine is associated with a lower risk of cardiac arrhythmia compared to terfenadine.

In a particular embodiment, the inhibitor of the invention is a third-generation H1 antihistamine chosen in the group consisting of: levocetirizine, desloratadine and fexofenadine, or a pharmaceutical salt or solvate thereof. Stereoisomers and conformers thereof are also herein encompassed.

In a particularly preferred embodiment, the inhibitor of the invention is hydroxyzine, cetirizine or levocetirizine.

In another particular embodiment, the inhibitor of the invention is chosen in the group consisting of: chlorodiphenhydramine, embramine, mirtazapine, olapatadine, phenindamine, phenyltoloxamine, pyrilamine, quetiapine, and trazodone.

The daily dose of the inhibitors of the invention to be administered to the infected subject is preferably the conventional dose used in the art and approved for said H1 antihistamine compounds.

This dose is for example comprised between about 12.5 mg/day and about 400 mg/day for hydroxyzine dose, when hydroxyzine is administered orally or through intramuscular injection.

In a preferred embodiment of the invention, the inhibitor of the invention is hydroxyzine which is administered orally at an effective dose comprised between about 12.5 mg/day and about 400 mg/day, preferably between about 25 mg/day and about 200 mg/day, preferably between about 50 mg/day and about 100 mg/day.

Alternatively, this dose is for example comprised between about 15 and 40 mg/ day for cetirizine dose, when cetirizine is used and is administered orally.

Preferably, low to moderate doses are used in the context of the invention, so as to minimize side effects of the antihistamine compound. As a matter of fact, low and moderate doses of antihistamine compound are generally well tolerated, especially when they are used on a short period, including in older adults who are the most prone to develop severe Covid-19 infection.

According to a preferred embodiment, the inhibitors of the invention in a general manner (anti H1 compound or other anti histamine compound) are administered to the subject at a dose comprised between about 1 mg/day and about 400 mg/day, preferably between about 5 mg/day and about 300 mg/day, preferably 12.5 mg/day and about 400 mg/day, preferably between about 25 mg/day and about 300 mg/day, more preferably between about 50 mg/day and about 200 mg/day and most preferably between about 50 mg/day and about 100 mg/day (in particular if said inhibitor is administered orally).

According to another preferred embodiment, the inhibitors of the invention are administered to the subject orally or by intramuscular perfusion at an effective dose comprised between about 1 mg/day and about 400 mg/day, preferably between about 5 mg/day and about 300 mg/day, preferably between about 12.5 mg and 400 mg per day, and preferably between 12.5 mg and 100 mg in older adults aged 70 years and over, and between 50 mg and 400 mg in younger adults aged 18 to 69 years old of age. These doses have to be divided by 2 to 4 folds if the patient furthermore suffers from renal impairment (<50mL/min) or liver failure.

It is within the skill of the person in the art to determine the desired therapeutic amount of the inhibitor of the invention to deliver by routine methods in the art.

The inhibitor of the invention is preferably incorporated in a pharmaceutical composition, said composition containing, apart from said inhibitor, a pharmaceutically acceptable excipient.

Said "pharmaceutically acceptable excipient" include for example water, saline, Ringer's solution, dextrose or other sugar solutions, Hank's solution, and other aqueous physiologically balanced salt solutions, phosphate buffer, bicarbonate buffer and Tris buffer.

The pharmaceutical composition of the invention may preferably be in a form suitable for the purposes of the invention. For example, said composition may be in a form suitable for parenteral or oral administration, such as a liquid suspension, or a solid dosage form (granules, pills, capsules or tablets). The term "parenteral" as used herein includes subcutaneous injection, intravenous, or intramuscular, injection.

The pharmaceutical composition of the invention is more preferably administered orally.

In a preferred embodiment, the agent of the invention is administered to the patient once it is diagnosed from severe COVID disease, and until the patient displays at least one symptom of the COVID disease. The treatment preferably ceases when the infection is over. Such short-term treatments are well tolerated and do not induce any side-effect or dependency in the treated patients.

### FIGURE LEGENDS

**Figure 1** describes the main characteristics of the Study cohort.
**Figure 2** shows kaplan-Meier curves for death in the full sample (A) (N=7,345) and in the matched analytic sample (B) (N=276) of patients who had been admitted to the hospital for Covid-19 according to hydroxyzine use. The shaded areas represent pointwise 95% confidence intervals.

### EXAMPLES

In this example part was examined the association between hydroxyzine use and the risk of death among adult patients who have been admitted to these medical centers with COVID-19, in time-to-event analyses adjusting for potential confounders, including patients' characteristics (sex, age, obesity, current smoking status, number of medical conditions associated with increased COVID-19-related mortality, any medication prescribed according to compassionate use or as part of a clinical trial, and the presence of current sleep or anxiety disorder), disease's severity at hospital admission (using markers of clinical and biological severity of COVID-19), and other psychotropic medications. Indeed, prior works have suggested that they may influence disease prognosis (including any benzodiazepine or Z-drug, any selective serotonin reuptake inhibitors (SSRI) antidepressant, any non-SSRI antidepressant, any mood stabilizer, and any antipsychotic medication). It was hypothesized that hydroxyzine use would be associated with reduced risk of death.

### 1. Material and Methods

### 1.1. Setting

This study was conducted at AP-HP, which comprises 39 hospitals, 23 of which are acute, 20 adult and 3 pediatric hospitals. Were included all adults aged 18 years or over who have been admitted with COVID-19 to these medical centers from the beginning of the epidemic in France, i.e. January 24th, until April 1st. COVID-19 was ascertained by a positive reverse-transcriptase-polymerase-chain-reaction (RT-PCR) test from analysis of nasopharyngeal or oropharyngeal swab specimens.

### 1.2. Data sources

The data from the AP-HP Health Data Warehouse ('Entrepôt de Données de Santé (EDS)') were used. This anonymized warehouse contains all the clinical data available on all inpatient visits for COVID-19 to any of the 39 Greater Paris University hospitals. The data obtained included patients' demographic characteristics, vital signs, laboratory test and RT-PCR test results, medication administration data, current medication lists, current diagnoses, and death certificates.

### 1.3. Variables assessed

The following data were assessed for each patient at the time of the hospitalization: sex; age, which was categorized based on the OpenSAFELY study results (i.e. 18-50, 51-70, 71-80, 81+); obesity, defined as having a body-mass index higher than 30 kg/m² or an International Statistical Classification of Diseases and Related Health Problems (ICD-10) diagnosis code for obesity (E66.0, E66.1, E66.2, E66.8, E66.9); self-reported current smoking status; any medical condition associated with increased COVID-19-related mortality based on ICD-10 diagnosis codes, including diabetes mellitus (E11), diseases of the circulatory system (100-199), diseases of the respiratory system (J00-J99), neoplasms (C00-D49), diseases of the blood and blood-forming organs and certain disorders involving the immune mechanism (D5-D8), delirium (F05, R41), and dementia (G30, G31, F01-F03); any sleep or anxiety disorder (G47*, F40-F48); any medication prescribed according to compassionate use or as part of a clinical trial (e.g. hydroxychloroquine, azithromycin, remdesivir, tocilizumab, sarilumab or dexamethasone); clinical severity of COVID-19 at admission, defined as having at least one of the following criteria: respiratory rate > 24 breaths/min or < 12 breaths/min, resting peripheral capillary oxygen saturation in ambient air < 90% , temperature > 40°C, or systolic blood pressure < 100 mm Hg; and biological severity of COVID-19 at admission, defined as having at least one of the following criteria: high neutrophil-to-lymphocyte ratio or low lymphocyte-to-C-reactive protein ratio (both variables were dichotomized at the median of the values observed in the full sample), or plasma lactate levels higher than 2 mmol/L; any prescribed psychotropic medication, including any benzodiazepine or Z-drug, any selective serotonin reuptake inhibitors (SSRI) antidepressant, any non-SSRI antidepressant, any mood stabilizer (i.e. lithium or antiepileptic medications with mood stabilizing effects), and any antipsychotic medication.

All medical notes and prescriptions are computerized in Greater Paris University hospitals. Medications and their mode of administration (i.e., dosage, frequency, date, condition of intake) were identified from medication administration data or scanned hand-written medical prescriptions, through two deep learning models based on BERT contextual embeddings, one for the medications and another for their mode of administration. The model was trained on the APmed corpus, a previously annotated dataset for this task. Extracted medications names were then normalized to the Anatomical Therapeutic Chemical (ATC) terminology using approximate string matching.

### 1.4. Hydroxyzine use

Study baseline was defined as the date of hospital admission. Hydroxyzine use was defined as receiving this medication at any time during the follow-up period, from study baseline to the end of the hospitalization or death.

### 1.5. Endpoint

The endpoint was the time from study baseline to death. Patients without an end-point event had their data censored on May 20th, 2020.

### 1.6. Statistical analysis

frequencies and means (± standard deviations (SD)) of each baseline characteristic described above were calculated in patients receiving or not receiving hydroxyzine and compared them using chi-square tests or Welch's t-tests.

To examine the associations between the use of hydroxyzine and the endpoint, Cox proportional-hazards regression models were performed. To help account for the nonrandomized prescription of hydroxyzine and reduce the effects of confounding, the primary analysis used propensity score analysis with inverse probability weighting. The individual propensities for receiving hydroxyzine were estimated using a multivariable logistic regression model that included patients' characteristics (sex, age, obesity, current smoking status, number of medical conditions associated with increased COVID-19-related mortality, the presence of current sleep or anxiety disorder, any medication prescribed according to compassionate use or as part of a clinical trial), disease's severity at hospital admission (using markers of clinical and biological severity of COVID-19), and other psychotropic medications (any benzodiazepine or Z-drug, any selective serotonin reuptake inhibitors (SSRI) antidepressant, any non-SSRI antidepressant, any mood stabilizer, and any antipsychotic medication). In the inverse-probability-weighted analyses, the predicted probabilities from the propensity-score models were used to calculate the stabilized inverse-probability-weighting weights. Associations between hydroxyzine use and the endpoint were then estimated using a multivariable Cox regression model including the inverse-probability-weighting weights. Kaplan-Meier curves were performed using the inverse-probability-weighting weights, and their pointwise 95% confidence intervals were estimated using the nonparametric bootstrap method.

Two sensitivity analyses were conducted, including a multivariable Cox regression model comprising as covariates the same variables as the inverse-probability-weighted analyses, and an univariate Cox regression model in a matched analytic samples using a 1:1 ratio, based on the same variables used for both the inverse-probability-weighted and the multivariable Cox regression analyses. Weighted Cox regression models were used when proportional hazards assumption was not met. To reduce the effects of confounding, optimal matching was used in order to obtain the smallest average absolute distance across all clinical characteristics between exposed patient and non-exposed matched controls.

Finally, within patients receiving hydroxyzine, the association of cumulative dose received (dichotomized by the median dose) with the endpoint was tested.

For all associations, residual analyses were performed to assess the fit of the data, check assumptions, including proportional hazards assumptions, and examined the potential influence of outliers. Statistical significance was fixed a *priori* at two-sided p-value<0.05. All analyses were conducted in R software version 2.4.3 (R Project for Statistical Computing).

### 2. Results

### 2.1. Characteristics of the cohort

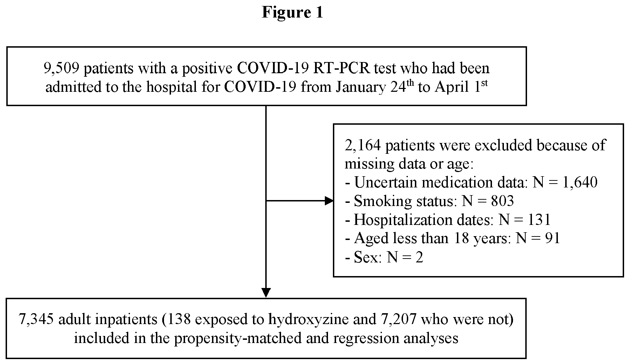

Of the 9,509 patients with a positive COVID-19 RT-PCR test consecutively admitted to the hospital, a total of 2,164 patients (22.8%) were excluded because of missing data (outside clinical and biological markers of severity) or their young age (i.e. less than 18 years old of age). Of the remaining 7,345 adult inpatients, 138 (1.9%) patients received hydroxyzine during the hospitalization, at a median daily dose of 25 mg (mean=49.8 mg, SD=51.5, minimum=12.5, maximum=300.0) per day (Figure 1).

COVID-19 RT-PCR test results were obtained after a mean delay of 5 days (SD=11.7, median=1 day) from the date of hospital admission. This delay was not significantly different between patients receiving or not receiving hydroxyzine [mean delay in the exposed group=7.1 day (SD=14.9); mean delay in the non-exposed group=5.0 day (SD=11.7); Welch's t-test=-1.63, p=0.106)].

Over a mean follow-up of 20.3 days (SD=27.5; median=7 days; range: 1 day to 117 days), 994 patients (13.5%) had an end-point event at the time of data cutoff on May 20th. Among patients receiving hydroxyzine, the mean follow-up was 22.4 days (SD=25.9; median=12.5 days; range: 1 day to 114 days), while it was of 20.2 days (SD=27.5; median=6 days; range: 4 day to 117 days) in those who were not (Welch's t-test=-0.97, p=0.336).

All baseline characteristics were independently and significantly associated with mortality, except for current smoking, any current sleep or anxiety disorder, any non-SSRI antidepressant, any mood stabilizer, and any antipsychotic medication (Table 1).

**Table 1. Associations of baseline clinical characteristics with the endpoint of death in the cohort of patients who had been admitted to the hospital for Covid-19 (N=7,345).**

| | | | | Endpoint of death | | |
|---|---|---|---|---|---|---|
| | Full population (N=7,345) | With the end-point event (N=994) | Without the end-point event (N=6,351) | Crude analysis | Mutivariable a nalysis | |
| | N (%) | N (%) | N (%) | HR (SE) / p-value | HR (SE) / p-value | Collinearity diagnosis (VIF) |
| Age | | | | | | 1.06 |
| *18 to 50 years* | 2709 (36.9%) | 42 (4.23%) | 2667 (42.0%) | Ref. | Ref. | |
| *51 to 70 years* | 2530 (34.4%) | 240 (24.1%) | 2290 (36.1%) | 5.31 (0.17) / <0.001* | 2.80 (0.19) / <0.001* | |
| *71 to 80 years* | 942 (12.8%) | 273 (27.5%) | 669 (10.5%) | 14.86 (0.17) / <0.001* | 7.51 (0.20) / <0.001* | |
| *More than 80 years* | 1164 (15.8%) | 439 (44.2%) | 725 (11.4%) | 16.99 (0.16) / <0.001* | 11.30 (0.19) / <0.001* | |
| Sex | | | | | | 1.06 |
| *Women* | 3619 (49.3%) | 359 (36.1%) | 3260 (51.3%) | Ref. | | |
| *Men* | 3726 (50.7%) | 635 (63.9%) | 3091 (48.7%) | 1.68 (0.07) / <0.001* | 1.32 (0.08) / <0.001* | |
| Obesity ^{α} | | | | | | 1.06 |
| | | | | | | |
| *Yes* | 975 (13.3%) | 216 (21.7%) | 759 (12.0%) | 1.51 (0.08) / <0.001* | 1.22 (0.09) / 0.024* | |
| *No* | 6370 (86.7%) | 778 (78.3%) | 5592 (88.0%) | Ref. | Ref. | |
| Smoking ^{β} | | | | | | 1.03 |
| | | | | | | |
| *Yes* | 623 (8.48%) | 149 (15.0%) | 474 (7.46%) | 1.51 (0.09) / <0.001* | 0.93 (0.10) / 0.447 | |
| *No* | 6722 (91.5%) | 845 (85.0%) | 5877 (92.5%) | Ref. | Ref. | |
| Any medical condition^{γ} | | | | | | 1.12 |
| | | | | | | |
| *0* | 4750 (64.7%) | 319 (32.1%) | 4431 (69.8%) | Ref. | Ref. | |
| *1* | 534 (7.27%) | 63 (6.34%) | 471 (7.42%) | 2.29 (0.14) / <0.001* | 1.78 (0.16) / <0.001* | |
| *2 or more* | 2061 (28.1%) | 612 (61.6%) | 1449 (22.8%) | 4.71 (0.07) / <0.001* | 3.50 (0.09) / <0.001* | |
| Medication according to compassionate use or as part of a clinical trial^{θ} | | | | | | 1.11 |
| | | | | | | |
| *Yes* | 1288 (17.5%) | 226 (22.7%) | 1062 (16.7%) | 1.13 (0.08) / 0.105 | 0.73 (0.09) / <0.001* | |
| *No* | 6057 (82.5%) | 768 (77.3%) | 5289 (83.3%) | Ref. | Ref. | |
| Anxiety or insomnia ^{€} | | | | | | 1.04 |
| | | | | | | |
| *Yes* | 299 (4.07%) | 101 (10.2%) | 198 (3.12%) | 2.38 (0.11) / <0.001* | 1.24 (0.12) / 0.067 | |
| *No* | 7046 (95.9%) | 893 (89.8%) | 6153 (96.9%) | Ref. | Ref. | |
| SSRI or SNRI | | | | | | 1.07 |
| | | | | | | |
| *Yes* | 257 (3.50%) | 68 (6.84%) | 189 (2.98%) | 1.53 (0.13) / 0.001* | 0.65 (0.13) / 0.001* | |
| *No* | 7088 (96.5%) | 926 (93.2%) | 6162 (97.0%) | Ref. | Ref. | |
| Any other antidepressant | | | | | | 1.06 |
| | | | | | | |
| *Yes* | 203 (2.76%) | 60 (6.04%) | 143 (2.25%) | 1.84 (0.13) / <0.001* | 0.83 (0.15) / 0.199 | |
| *No* | 7142 (97.2%) | 934 (94.0%) | 6208 (97.7%) | Ref. | Ref. | |
| Any mood stabilizer medication ^{Ω} | | | | | | 1.05 |
| | | | | | | |
| *Yes* | 287 (3.91%) | 69 (6.94%) | 218 (3.43%) | 1.42 (0.12) / 0.005* | 0.90 (0.14) / 0.444 | |
| *No* | 7058 (96.1%) | 925 (93.1%) | 6133 (96.6%) | Ref. | Ref. | |
| Any benzodiazepine or Z-drug | | | | | | 1.14 |
| | | | | | | |
| *Yes* | 752 (10.2%) | 268 (27.0%) | 484 (7.62%) | 2.53 (0.07) / <0.001* | 1.56 (0.09) / <0.001* | |
| *No* | 6593 (89.8%) | 726 (73.0%) | 5867 (92.4%) | Ref. | Ref. | |
| Any antipsychotic | | | | | | 1.07 |
| | | | | | | |
| *Yes* | 264 (3.59%) | 60 (6.04%) | 204 (3.21%) | 1.26 (0.13) / 0.086 | 0.81 (0.14) / 0.131 | |
| *No* | 7081 (96.4%) | 934 (94.0%) | 6147 (96.8%) | Ref. | Ref. | |
| Clinical severity of Covid-19 at admission ^{µ} | | | | | | 1.15 |
| | | | | | | |
| *Yes* | 1564 (21.3%) | 421 (42.4%) | 1143 (18.0%) | 1.81 (0.08) / <0.001* | 1.69 (0.09) / <0.001* | |
| *No* | 1858 (25.3%) | 265 (26.7%) | 1593 (25.1%) | Ref. | Ref. | |
| *Missing* | 3923 (53.4%) | 308 (31.0%) | 3615 (56.9%) | 0.59 (0.08) / <0.001* | 1.66 (0.11) / <0.001* | |
| Biological severity of Covid-19 at admission ^{κ} | | | | | | 1.15 |
| | | | | | | |
| *Yes* | 2439 (33.2%) | 592 (59.6%) | 1847 (29.1%) | 1.94 (0.08) / <0.001* | 1.50 (0.09) / <0.001* | |
| *No* | 1861 (25.3%) | 257 (25.9%) | 1604 (25.3%) | Ref. | Ref. | |
| *Missing* | 3045 (41.5%) | 145 (14.6%) | 2900 (45.7%) | 0.39 (0.1) / <0.001* | 0.75 (0.12) / <0.020* | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Smoking status was self-reported. ^{γ} Assessed using ICD-10 diagnosis codes for diabetes mellitus (E11), diseases of the circulatory system (I00-I99), diseases of the respiratory system (J00-J99), neoplasms (C00-D49), diseases of the blood and blood-forming organs and certain disorders involving the immune mechanism (D5-D8), delirium (F05, R41) and dementia (G30, G31, F01-F03). ^{θ} Any medication prescribed as part of a clinical trial or according to compassionate use (e.g., hydroxychloroquine, azithromycin, remdesivir, tocilizumab, sarilumab or dexamethasone). ^{€} Assessed using ICD-10 diagnosis codes for anxiety, dissociative, stress-related, somatoform and other nonpsychotic mental disorders (F40-F48) and insomnia (G47). ^{Ω} Included lithium or antiepileptic medications with mood stabilizing properties. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{κ} Defined as having at least one of the following criteria: high neutrophil-to-lymphocyte ratio, low *lymphocyte-to-C-reactive protein* (both variables were dichotomized at the median of the values observed in the full sample), and plasma *lactate* levels *higher than 2 mmol*/*L.* * p-value is significant (p<0.05). Abbreviations: HR, hazard ratio; SE, standard error; VIF, variance inflation factor. | | | | | | |

The distribution of patients' characteristics according to hydroxyzine use is shown in Table 2. In the full sample, hydroxyzine use significantly differed according to all baseline characteristics (Table 2). The direction of these associations indicated older age and overall greater medical severity of patients receiving hydroxyzine than those who did not. After applying the propensity score weights, these differences were substantially reduced (Table 2). In the matched analytic sample, there were no significant differences in patients' characteristics according to hydroxyzine use (Table 2).

**Table 2. Characteristics of patients with COVID-19 receiving or not receiving hydroxyzine.**

| | Exposed to hydroxyzine (N=138) | Not exposed to hydroxyzine (N=7,207) | Non-exposed matched group (N=138) | Exposed to hydroxyzine | Exposed to hydroxyzine | Exposed to hydroxyzine |
|---|---|---|---|---|---|---|
| | | | | vs. | vs. | vs. |
| | | | | Not exposed to hydroxyzine | Not exposed to hydroxyzine | Non-exposed matched group |
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| Age | | | | 13.97 (0.003*) | 2.90 (0.408) | 1.13 (0.771) |
| *18 to 50 years* | 35 (25.4%) | 2674 (37.1%) | 28 (20.3%) | | | |
| *51 to 7 years* | 59 (42.8%) | 2471 (34.3%) | 61 (44.2%) | | | |
| *71 to 80 years* | 27 (19.6%) | 915 (12.7%) | 29 (21.0%) | | | |
| *More than 80 years* | 17 (12.3%) | 1147 (15.9%) | 20 (14.5%) | | | |
| Sex | | | | 6.21 (0.013*) | 1.96 (0.162) | 0.02 (0.901) |
| *Women* | 53 (38.4%) | 3566 (49.5%) | 51 (37.0%) | | | |
| *Men* | 85 (61.6%) | 3641 (50.5%) | 87 (63.0%) | | | |
| Obesity ^{α} | | | | 14.79 (<0.001*) | 5.25 (0.022*) | 0.52 (0.471) |
| | | | | | | |
| *Yes* | 34 (24.6%) | 941 (13.1%) | 28 (20.3%) | | | |
| *No* | 104 (75.4%) | 6266 (86.9%) | 110 (79.7%) | | | |
| Smoking ^{β} | | | | 11.09 (0.001*) | 3.67 (0.055) | 0.03 (0.869) |
| | | | | | | |
| *Yes* | 23 (16.7%) | 600 (8.33%) | 21 (15.2%) | | | |
| *No* | 115 (83.3%) | 6607 (91.7%) | 117 (84.8%) | | | |
| Any medical condition ^{γ} | | | | 84.95 (<0.001*) | 33.78 (<0.001*) | 0.18 (0.912) |
| | | | | | | |
| *0* | 40 (29.0%) | 4710 (65.4%) | 41 (29.7%) | | | |
| *1* | 13 (9.42%) | 521 (7.23%) | 11 (7.97%) | | | |
| *2 or more* | 85 (61.6%) | 1976 (27.4%) | 86 (62.3%) | | | |
| Medication according to compassionate use or as part of a clinical trial ^{θ} | | | | 40.9 (<0.001*) | 10.94 (0.001*) | <0.01 (>0.99) |
| *Yes* | 53 (38.4%) | 1235 (17.1%) | 54 (39.1%) | | | |
| *No* | 85 (61.6%) | 5972 (82.9%) | 84 (60.9%) | | | |
| Anxiety or insomnia ^{€} | | | | 26.7 (<0.001*) | 14.32 (<0.001*) | 0.03 (0.855) |
| | | | | | | |
| *Yes* | 18 (13.0%) | 281 (3.90%) | 16 (11.6%) | | | |
| *No* | 120 (87.0%) | 6926 (96.1%) | 122 (88.4%) | | | |
| SSRI or SNRI | | | | 9.73 (0.002*) | 1.12 (0.290) | <0.01 (>0.99) |
| | | | | | | |
| *Yes* | 12 (8.70%) | 245 (3.40%) | 12 (8.70%) | | | |
| *No* | 126 (91.3%) | 6962 (96.6%) | 126 (91.3%) | | | |
| Any other antidepressant | | | | 16.23 (<0.001*) | 2.45 (0.117) | <0.01 (>0.99) |
| | | | | | | |
| *Yes* | 12 (8.70%) | 191 (2.65%) | 12 (8.70%) | | | |
| *No* | 126 (91.3%) | 7016 (97.3%) | 126 (91.3%) | | | |
| Any mood stabilizer medication ^{Ω} | | | | 20.10 (<0.001*) | 5.16 (0.023*) | <0.01 (>0.99) |
| | | | | | | |
| *Yes* | 16 (11.6%) | 271 (3.76%) | 16 (11.6%) | | | |
| *No* | 122 (88.4%) | 6936 (96.2%) | 122 (88.4%) | | | |
| Any benzodiazepine or Z-drug | | | | 69.32 (<0.001*) | 26.21 (<0.001*) | <0.01 (>0.99) |
| | | | | | | |
| *Yes* | 44 (31.9%) | 708 (9.82%) | 45 (32.6%) | | | |
| *No* | 94 (68.1%) | 6499 (90.2%) | 93 (67.4%) | | | |
| Any antipsychotic | | | | 65.57 (<0.001*) | 24.29 (<0.001*) | <0.01 (>0.99) |
| | | | | | | |
| *Yes* | 23 (16.7%) | 241 (3.34%) | 23 (16.7%) | | | |
| *No* | 115 (83.3%) | 6966 (96.7%) | 115 (83.3%) | | | |
| Clinical severity of Covid-19 at admission ^{µ} | | | | 48.7 (<0.001*) | 19.13 (<0.001*) | 0.17 (0.917) |
| | | | | | | |
| *Yes* | 53 (38.4%) | 1511 (21.0%) | 50 (36.2%) | | | |
| *No* | 51 (37.0%) | 1807 (25.1%) | 54 (39.1%) | | | |
| *Missing* | 34 (24.6%) | 3889 (54.0%) | 34 (24.6%) | | | |
| | | | | | | |
| Biological severity of Covid-19 at admission ^{κ} | | | | 29.65 (<0.001*) | 3.52 (0.172) | 0.94 (0.626) |
| *Yes* | 69 (50.0%) | 2370 (32.9%) | 74 (53.6%) | | | |
| *No* | 42 (30.4%) | 1819 (25.2%) | 43 (31.2%) | | | |
| *Missing* | 27 (19.6%) | 3018 (41.9%) | 21 (15.2%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Smoking status was self-reported. ^{γ} Assessed using ICD-10 diagnosis codes for diabetes mellitus (E11), diseases of the circulatory system (I00-I99), diseases of the respiratory system (J00-J99), neoplasms (C00-D49), diseases of the blood and blood-forming organs and certain disorders involving the immune mechanism (D5-D8), delirium (F05, R41) and dementia (G30, G31, F01-F03). ^{θ} Any medication prescribed as part of a clinical trial or according to compassionate use (e.g., hydroxychloroquine, azithromycin, remdesivir, tocilizumab, sarilumab or dexamethasone). ^{€} Assessed using ICD-10 diagnosis codes for anxiety, dissociative, stress-related, somatoform and other nonpsychotic mental disorders (F40-F48) and insomnia (G47). ^{Ω} Included lithium or antiepileptic medications with mood stabilizing properties. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{κ} Defined as having at least one of the following criteria: high neutrophil-to-lymphocyte ratio, low *lymphocyte-to-C-reactive protein* (both variables were dichotomized at the median of the values observed in the full sample), and plasma *lactate* levels *higher than 2 mmol*/*L.* * p-value is significant (p<0.05). Abbreviations: HR, hazard ratio; SE, standard error. | | | | | | |

### 2.2. Study endpoint

Among patients receiving hydroxyzine, death occurred in 15 patients (10.9%), while 979 non-exposed patients (16.6%) had this outcome (Table 3). Unadjusted hazard ratio of the association between hydroxyzine use and the endpoint of death was not significant (HR, 0.60, 95% CI, 0.36 to 1.00, p=0.051) (Table 3).

**Table 3. Association between hydroxyzine use and the endpoint of death in the full sample and in the matched analytic sample.**

| | **Number of events / Number of patients** | **Crude Cox regression analysis** | **Multivariable Cox regression analysis** | **Analysis weighted by inverse-probability-weighting weights** | **Number of events / Number of patients** | **Univariate Cox regression in the matched analytic sample (1:1)** |
|---|---|---|---|---|---|---|
| | N (%) | HR (95% CI; p-value) | HR (95% CI; p-value) | HR (95% Cl; p-value) | N (%) | HR (95% CI; p-value) |
| No hydroxyzine | 979 / 7,207 (16.6%) | Ref. | Ref. | Ref. | 29/138 (21.0%) | Ref. |
| Hydroxyzine | 15/138 (10.9%) | 0.60 (0.36 - 1.00; 0.051) | 0.42 (0.25 - 0.71; 0.001*) | 0.40 (0.24 - 0.68; 0.001*) | 15/138 (10.9%) | 0.37 (0.20 - 0.69; 0.002*) |

| | | | | | | |
|---|---|---|---|---|---|---|
| * p-value is significant (p<0.05). | | | | | | |

However, the primary multivariable analysis with inverse probability weighting taking into account differences in baseline characteristics showed a significant association between hydroxyzine use and reduced risk of death (HR, 0.40; 95% CI, 0.24 to 0.68, p=0.001) (Table 3, Figure 2).

In sensitivity analyses, the multivariable Cox regression model in the full sample yielded similar results (HR, 0.42; 95% CI, 0.25 to 0.71, p=0.001), as did the univariate Cox regression model in the matched analytic sample (HR, 0.37; 95% CI, 0.20 to 0.69, p=0.002) (Table 3, Figure 2).

Finally, exposure to higher (mean=2908.6 mg, median=1250 mg, SD=2317.2, median daily dose=75 mg, SD=63.6) rather than lower (mean=202.6, median=175 mg, SD=126.9, median daily dose=25 mg, SD=4.2) cumulative doses of hydroxyzine was significantly associated with lower risk of death (Table 4).

**Table 4. Associations of hydroxyzine dose with the endpoint of death among patients using hydroxyzine.**

| | **Number of events / Number of patients** | **Crude Cox regression analysis** | **Cox regression adjusted for age and sex** | **Multivariable Cox regression analysis** |
|---|---|---|---|---|
| | N (%) | HR (95% CI; p-value) | HR (95% CI; p-value) | HR (95% CI; p-value) |
| ***Cumulative dose*** | | | | |
| Low doses | 8/53 (15.1%) | Ref. | Ref. | Ref. |
| High doses | 2/53 (3.8%) | 0.10 (0.02 - 0.48; 0.004*) | 0.12 (0.03 - 0.57; 0.007*) | 0.10 (0.02 - 0.45; 0.003*) |

| | | | | |
|---|---|---|---|---|
| * p-value is significant (p<0.05). The variable cumulative dose has been dichotomized by the median. | | | | |

A post-hoc analysis indicated that in the full sample, we had 80% power to detect hazard ratios for hydroxyzine of at least 1.93/0.32 and 2.54/0.18 for the endpoint of death in the full sample and in the matched analytic sample, respectively.

### 3. Discussion

In this multicenter retrospective observational study involving a large sample of patients admitted to the hospital with COVID-19, it was found that hydroxyzine use, at a median daily dose of 25 mg (mean 49.8 mg, SD=51.5), was significantly and substantially associated with reduced risk of death, independently of patients' characteristics, clinical and biological markers of disease's severity at hospital admission, and use of other psychotropic medications. That association was significantly stronger at higher rather than lower cumulative doses.

In the analyses, the effects of confounding were minimized in several different ways. First, a multivariable regression model was used, with inverse probability weighting to minimize the effects of confounding by indication. Sensitivity analyses were also performed, including a multivariable Cox regression models and an univariate Cox regression model in a matched analytic sample, that showed similar results. Second, although some amount of unmeasured confounding may remain, the present analyses adjusted for numerous potential confounders, including patients' characteristics (sex, age, obesity, current smoking status, number of medical conditions associated with increased COVID-19-related mortality, any medication prescribed according to compassionate use or as part of a clinical trial, and the presence of current sleep or anxiety disorder), disease's severity at hospital admission (using markers of clinical and biological severity of COVID-19), and other psychotropic medications as prior work have suggested that they may influence disease prognosis (including any benzodiazepine or Z-drug, any selective serotonin reuptake inhibitors (SSRI) antidepressant, any non-SSRI antidepressant, any mood stabilizer, and any antipsychotic medication).

In this multicenter observational retrospective study involving patients admitted to the hospital for COVID-19, hydroxyzine use during the hospitalization was significantly and substantially associated with lower risk of death, independently of patients' characteristics, clinical and biological markers of disease's severity at hospital admission, and use of other psychotropic medications. This association might be explained by the known anti-inflammatory properties of H1 antihistamines.

### BIBLIOGRAPHIC REFERENCES

1. Anderson RM, Heesterbeek H, Klinkenberg D, Hollingsworth TD. How will country-based mitigation measures influence the course of the COVID-19 epidemic? Lancet. Mar 21 2020;395(10228):931-934.
2. Beigel JH, Tomashek KM, Dodd LE, et al. Remdesivir for the Treatment of Covid-19 - Preliminary Report. New England Journal of Medicine. 2020.
3. Gordon DE, Jang GM, Bouhaddou M, et al. A SARS-CoV-2 protein interaction map reveals targets for drug repurposing. Nature. 2020:1-13.
4. Chevance A, Gourion D, Hoertel N, et al. Ensuring mental health care during the SARS-CoV-2 epidemic in France: a narrative review. L'encephale. 2020.
5. Stebbing J, Phelan A, Griffin I, et al. COVID-19: combining antiviral and anti-inflammatory treatments. The Lancet Infectious Diseases. 2020;20(4):400-402.
6. Liu Y, Yan L-M, Wan L, et al. Viral dynamics in mild and severe cases of COVID-19. The Lancet Infectious Diseases. 2020.
7. New insights into the pathophysiology of allergic rhinitis. Allergy & Asthma Proceedings; 2007.
8. Mandhane SN, Shah JH, Bahekar PC, et al. Characterization of anti-inflammatory properties and evidence for no sedation liability for the novel antihistamine SUN-1334H. International archives of allergy and immunology 2010;151(1):56-69.
9. Liu T, Zhang L, Joo D, et al. NF-κB signaling in inflammation. Signal transduction and targeted therapy 2017;2(1):1-9.
10. Hoertel N, SANCHEZ RICO M, VERNET R, et al. Association between SSRI Antidepressant Use and Reduced Risk of Intubation or Death in Hospitalized Patients with Coronavirus Disease 2019: a Multicenter Retrospective Observational Study. medRxiv 2020:2020.07.09.20143339.
11. Williamson E, Walker AJ, Bhaskaran KJ, et al. OpenSAFELY: factors associated with COVID-19-related hospital death in the linked electronic health records of 17 million adult NHS patients. MedRxiv 2020.
12. Zhou F, Yu T, Du R, et al. Clinical course and risk factors for mortality of adult inpatients with COVID-19 in Wuhan, China: a retrospective cohort study. The Lancet 2020.
13. Ruan Q, Yang K, Wang W, et al. Clinical predictors of mortality due to COVID-19 based on an analysis of data of 150 patients from Wuhan, China. Intensive care medicine 2020:1-3.
14. Hur K, Price CP, Gray EL, et al. Factors Associated With Intubation and Prolonged Intubation in Hospitalized Patients With COVID-19. Otolaryngology--Head and Neck Surgery.
15. Haut Conseil de la Santé Publique. Statement on the management at home or in a care facility of suspected or confirmed Covid-19 patients. April 8, 2020. Statement on the management at home or in a care facility of suspected or confirmed Covid-19 patients.
16. Lagunas-Rangel FA. Neutrophil-to-lymphocyte ratio and lymphocyte-to-C-reactive protein ratio in patients with severe coronavirus disease 2019 (COVID-19): A meta-analysis. Journal of medical virology 2020.
17. Devlin J, Chang M-W, Lee K, et al. Bert: Pre-training of deep bidirectional transformers for language understanding. arXiv preprint arXiv: 181004805 2018.
18. Jouffroy J, Feldman SF, Lerner I, et al. MedExt: combining expert knowledge and deep learning for medication extraction from French clinical texts.
19. Robins JM, Hernan MA, Brumback B. Marginal structural models and causal inference in epidemiology: LWW, 2000.
20. Geleris J, Sun Y, Platt J, et al. Observational study of hydroxychloroquine in hospitalized patients with Covid-19. New England Journal of Medicine 2020.
21. Kaplan EL, Meier P. Nonparametric estimation from incomplete observations. Journal of the American statistical association 1958;53(282):457-81.
22. Efron B. Nonparametric standard errors and confidence intervals. canadian Journal of Statistics 1981;9(2): 139-58.
23. Zappia, C., Granja-Galeano, G., Fernández, N. et al. Effects of histamine H1 receptor signaling on glucocorticoid receptor activity. Role of canonical and non-canonical pathways. Sci Rep 5, 17476 (2015).
24. Antonela Diaz Nebreda, Carlos Daniel Zappia, Angela Rodriguez Gonzalez, Ana Sahores, Máximo Sosa, Valeria Burghi, Federico Monczor, Carlos Davio, Natalia Fernández, Carina Shayo, Involvement of histamine H1 and H2 receptor inverse agonists in receptor's crossregulation, European Journal of Pharmacology, Volume 847, 2019, Pages 42-52, ISSN 0014-2999

## Claims

1. An inhibitor of the histamine H1 receptor for use for treating a viral infection due to a Severe Acute Respiratory Syndrome-related coronavirus (SARS-CoV), in particular SARS-CoV-2, in a patient in need thereof.

2. The histamine H1 receptor inhibitor for use according to claim 1, for treating patients suffering from a symptomatic COVID-19 disease, advantageously from a strong or severe symptomatic COVID-19 disease.

3. The histamine H1 receptor inhibitor for use according to any one of claims 1 to 2, for preventing and/or treating acute respiratory distress syndrome associated to said viral infection.

4. The histamine H1 receptor inhibitor for use according to any one of claims 1 to 3 for lowering the risk of intubation, for lowering the risk of hospitalization or for increasing the survival rate of patients infected by said coronavirus.

5. The histamine H1 receptor inhibitor for use according to any one of claims 1 to 4, said inhibitor being a first generation antihistamine compound chosen in the group consisting of: mepyramine, chloropyramine, antazoline, tripelennamine, diphenhydramine, carbinoxamine, doxylamine, orphenadrine, bromazine, clemastine, dimenhydrinate, pheniramine, chlorpheniramine, dexchlorpheniramine, dexbrompheniramine, brompheniramine, triprolidine, dimetindene, buclizine, cinnarizine, cyclizine, etodroxizine, chlorcyclizine, hydroxyzine, pipoxizine, meclizine, promethazine, alimemazine, cyproheptadine, setastine or a pharmaceutical salt or solvate thereof.

6. The histamine H1 receptor inhibitor for use according to any one of claims 1 to 5, said inhibitor having a diphenyl methyl piperazine structure, preferably chosen in the group consisting of: meclizine, cyclizine, cetirizine, buclizine, cinnarizine, etodroxizine, chlorcyclizine, and hydroxyzine or a pharmaceutical salt or solvate thereof.

7. The histamine H1 receptor inhibitor for use according to any one of claims 1 to 5, said inhibitor being hydroxyzine or a pharmaceutical salt or solvate thereof.

8. The histamine H1 receptor inhibitor for use according to any one of claims 1 to 5, wherein said inhibitor is a second-generation H1 antihistamine chosen in the group consisting of: astemizole, ketotifen, cetirizine, loratadine, desloratadine, rupatadine, mizolastine, acrivastine, ebastine, bilastine, bepotastine, terfenadine, quifenadine, azelastine, levocabastine, olopatadine, or a pharmaceutical salt or solvate thereof

9. The histamine H1 receptor inhibitor for use according to any one of claims 1 to 5, wherein said inhibitor is cetirizine or a pharmaceutical salt or solvate thereof.

10. The histamine H1 receptor inhibitor for use according to any one of claims 1 to 5, wherein said inhibitor is a third-generation H1 antihistamine chosen in the group consisting of: levocetirizine, desloratadine and fexofenadine.

11. The histamine H1 receptor inhibitor for use according to any one of claims 1 to 5, wherein said inhibitor is hydroxyzine, cetirizine, or levocetirizine.

12. The histamine H1 receptor inhibitor for use according to any one of claims 1 to 5, wherein said inhibitor is chosen in the group consisting of : chlorodiphenhydramine, embramine, mirtazapine, olapatadine, phenindamine, phenyltoloxamine, pyrilamine, quetiapine, and trazodone.

13. The histamine H1 receptor inhibitor for use according to any of claim 1 to 12, wherein said inhibitor is administered at an effective dose comprised between about 1 mg/day and about 400 mg/day, preferably between about 5 mg/day and about 300 mg/day.

14. The histamine H1 receptor inhibitor for use according to claim 7, wherein said inhibitor is hydroxyzine which is administered orally at an effective dose comprised between about 12.5 mg/day and about 400 mg/day, preferably between about 25 mg/day and about 100 mg/day.
